# EUROPEAN PATENT SPECIFICATION

(11) **EP 2 382 472 B1**
(45) Date of publication and mention of the grant of the patent: **06.05.2015**
(21) Application number: 09795663.5
(22) Date of filing: 22.12.2009
(51) Int. Cl.: G01N 33/574

(54) **METHODS AND COMPOSITIONS FOR DIAGNOSTIC USE IN CANCER PATIENTS**
VERFAHREN UND ZUSAMMENSETZUNGEN ZUR DIAGNOSTISCHEN VERWENDUNG BEI KREBSPATIENTEN
PROCÉDÉS ET COMPOSITIONS POUR UNE UTILISATION DIAGNOSTIQUE CHEZ LES PATIENTS ATTEINTS DE CANCER

(30) Priority: 23.12.2008 US 140392 P
(43) Date of publication of application: 02.11.2011
(73) Proprietor: F.Hoffmann-La Roche AG, 4070 Basel (CH)
(72) Inventor: YAN, Yibing, San Francisco, CA 94116 (US); FIELDER, Paul, J., Emerald Hills, CA 94062 (US); WU, Qun, Jenny, Belmont, CA 94002 (US)
(74) Representative: Brodbeck, Michel
(86) International application number: PCT/US2009/069254
(87) International publication number: WO 2010/075420

(56) References cited:
- WEI S -C ET AL N ET AL: "Placenta growth factor expression is correlated with survival of patients with colorectal cancer" GUT, vol. 54, no. 5, May 2005 (2005-05), pages 666-672, XP002569992 ISSN: 0017-5749
- PARR C ET AL: "Placenta growth factor is over-expressed and has prognostic value in human breast cancer" EUROPEAN JOURNAL OF CANCER, PERGAMON PRESS, OXFORD, GB, vol. 41, no. 18, 1 December 2005 (2005-12-01), pages 2819-2827, XP025298153 ISSN: 0959-8049 [retrieved on 2005-12-01]
- TAYLOR ALICE P ET AL: "Altered tumor vessel maturation and proliferation in placenta growth factor-producing tumors: Potential relationship to post-therapy tumor angiogenesis and recurrence." INTERNATIONAL JOURNAL OF CANCER, vol. 105, no. 2, 10 June 2003 (2003-06-10) , pages 158-164, XP002569993 ISSN: 0020-7136
- JAIN RAKESH K ET AL: "alpha PIGF: A new kid on the antiangiogenesis block" CELL, vol. 131, no. 3, November 2007 (2007-11), pages 443-445, XP002569994 ISSN: 0092-8674
- FISCHER CHRISTIAN ET AL: "Anti-PlGF inhibits growth of VEGF(R)-inhibitor-resistant tumors without affecting healthy vessels." CELL 2 NOV 2007, vol. 131, no. 3, 2 November 2007 (2007-11-02), pages 463-475, XP002569995 ISSN: 0092-8674
- FERRARA N ET AL: "Discovery and development of bevacizumab, an anti-VEGF antibody for treating cancer" NATURE REVIEWS. DRUG DISCOVERY, NATURE PUBLISHING GROUP, BASINGSTOKE, GB, vol. 3, no. 5, 1 May 2004 (2004-05-01), pages 391-400, XP002302380 ISSN: 1474-1784

## Description

### Field of the Invention

The present invention relates to methods and compositions useful for predicting clinical outcome and for monitoring cancer patients treated with anti-angiogenic therapy.

### Background of the Invention

Cancer is one of the most deadly threats to human health. In the U.S. alone, cancer affects nearly 1.3 million new patients each year, and is the second leading cause of death after cardiovascular disease, accounting for approximately 1 in 4 deaths. Solid tumors are responsible for most of those deaths. Although there have been significant advances in the medical treatment of certain cancers, the overall 5-year survival rate for all cancers has improved only by about 10% in the past 20 years. Cancers, or malignant tumors, metastasize and grow rapidly in an uncontrolled manner, making timely detection and treatment extremely difficult.

Depending on the cancer type, patients typically have several treatment options available to them including chemotherapy, radiation and antibody-based drugs. Diagnostic methods useful for predicting clinical outcome from the different treatment regimens would greatly benefit clinical management of these patients. Several studies have explored the correlation of gene expression with the identification of specific cancer types, e.g., by mutation-specific assays, microarray analysis, qPCR, etc. Such methods may be useful for the identification and classification of cancer presented by a patient. However, much less is known about the predictive or prognostic value of gene expression with clinical outcome.

Thus, there is a need for objective, reproducible methods for predicting treatment outcome such as progression free survival of cancer patients or for monitoring the progress of such treatment and thereby selecting the optimal treatment regimen for each patient.

Wei SC et al., Gut. 2005 May;54(5):666-72 discloses that the PlGF expression level is significantly higher in tumour than in non-tumour tissues. Parr C et al., Eur J Cancer. 2005 Dec;41(18):2819-27. Epub 2005 Nov 4 discloses that PlGF is over-expressed in breast cancer tissues. Taylor AP et al, Int J Cancer. 2003 Jun 10;105(2):158-64 describes the role of PlGF in rapid restoration of tumor blood supply after treatment and thus to enhanced likelihood of tumor regrowth.

### Summary of the Invention

The methods of the present invention can be utilized in a variety of settings, including, for example, in aiding in methods of treating cancer patients or in patient selection during the course of drug development, prediction of likelihood of success when treating an individual patient with a particular treatment regimen, in assessing disease progression, in monitoring treatment efficacy, in determining prognosis for individual patients and in assessing predisposition of an individual to benefit from a particular anti-cancer therapy.

The present invention is based, in part, on the discovery that expression levels of certain biomarkers in patients suffering from cancer correlate with reduced clinical benefit from anti-angiogenic therapy alone. Accordingly, in one aspect the invention provides a method of optimizing therapeutic efficacy for the treatment of cancer, comprising the step of detecting the expression level of placental growth factor (PlGF) in a sample obtained from the patient wherein increased expression of PlGF in the sample as compared to a reference sample indicates that the patient may benefit from anti-cancer therapy other than or in addition to anti-angiogenic therapy. In some embodiments, the method further comprises administering an anti-cancer therapy to the patient, wherein the anti-cancer therapy is other than or in addition to the anti-angiogenic therapy.

In another aspect the invention provides a method of identifying a cancer patient who may benefit from anti-cancer therapy other than or in addition to anti-angiogenic therapy, comprising the step of detecting the expression level of placental growth factor (PlGF) in a sample obtained from the patient wherein increased expression of PlGF in the sample as compared to a reference sample indicates that the patient may benefit from anti-cancer therapy other than or in addition to anti-angiogenic therapy. In one embodiment, the sample from the patient is obtained before or at commencement of the anti-angiogenic therapy. In one embodiment, the sample from the patient is obtained after commencement of the anti-angiogenic therapy. In some embodiments, the method further comprises administering an anti-cancer therapy to the patient, wherein the anti-cancer therapy is other than or in addition to the anti-angiogenic therapy.

In another aspect the invention provides a method of predicting responsiveness of a cancer patient to anti-angiogenic therapy comprising determining the expression level of PlGF in a sample obtained from the patient wherein increased expression levels of PlGF as compared to a reference sample indicates that the patient is less likely to be responsive to the anti-angiogenic therapy alone. In one embodiment, the sample from the patient is obtained before or at commencement of the anti-angiogenic therapy. In one embodiment, the sample from the patient is obtained after commencement of the anti-angiogenic therapy. In some embodiments, the method further comprises administering an anti-cancer therapy to the patient, wherein the anti-cancer therapy is other than or in addition to the anti-angiogenic therapy.

The invention also provides a method of treating a patient with cancer comprising administering to the patient an anti-cancer therapy other than or in addition to anti-angiogenic therapy, wherein a sample obtained from the patient shows increased expression levels of PlGF as compared to a reference sample. In one embodiment, the sample from the patient is obtained before or at commencement of the anti-angiogenic therapy. In one embodiment, the sample from the patient is obtained after commencement of the anti-angiogenic therapy. The sample may be obtained from the patient before commencement of treatment. In one embodiment the sample has a PlGF expression level of greater than or equal to 28 pg/ml. In some embodiments the sample may be obtained from a patient currently being treated with anti-angiogenic therapy. In one embodiment, the sample from the patient is obtained after commencement of the anti-angiogenic therapy. In one embodiment the sample has a PlGF expression level of greater than or equal to 50 pg/ml.

In another aspect the invention provides a method of treating a cancer patient comprising administering to the patient an anti-angiogenic therapy, wherein a sample obtained from the patient shows low or decreased expression levels of PlGF as compared to a reference sample. The sample may be obtained from the patient before commencement of treatment. In one embodiment the sample has a PlGF expression level of less than or equal to 28 pg/ml. In some embodiments the sample may be obtained from a patient currently being treated with anti-angiogenic therapy. In one embodiment, the sample from the patient is obtained after commencement of the anti-angiogenic therapy. In one embodiment the sample has a PlGF expression level of less than or equal to 50 pg/ml. In some embodiments, the anti-angiogenic therapy comprises administration of an anti-VEGF antibody, e.g., bevacizumab.

In any of the methods described herein, the sample may be a tissue or cell sample or obtained from blood, plasma and/or serum. In certain embodiments the sample is obtained from the patient before commencement of treatment of the cancer. In certain embodiments the sample is obtained from the patient after commencement of treatment of the cancer. For example, the sample is obtained within 24 hours after beginning cancer treatment or within 2, 3, 5, 10, 14, 20, 25, 28, 42, or 56 days after commencement of cancer treatment.

In any of the methods described herein, expression levels of the one or more genes or gene products can be determined at the nucleic acid level, protein level or secretion or surface expression level of the protein. In certain embodiments the increase in expression levels of PlGF is an increase of 1.4-1.8 fold in expression level. In some embodiments the increase in expression levels of PlGF is an increase of at least 1.5 fold in expression level.

In some embodiments of the methods of the invention, the cancer is carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors) or Meigs' syndrome.

In one embodiment the cancer is breast cancer, including, e.g., metastatic breast cancer.

The methods of the invention can be performed with any anti-cancer agent described herein below. The anti-angiogenic agent may be any of the anti-angiogenesis agents described herein below, alone or in combination. In some embodiments the anti-angiogenic therapy comprises administration of VEGF antagonist, including, e.g., an anti-VEGF antibody. In some embodiments, the anti-VEGF antibody is bevazicumab.

Any embodiment described herein or any combination thereof applies to any and all methods of the invention described herein.

### Brief Description of the Figures

**Figure 1** **is** a graph comparing overall survival of patients with breast cancer to plasma PlGF levels at Day 0 (before commencement of treatment).
**Figure 2** is a graph showing that plasma PlGF levels increased in response to treatment with bevacizumab.
**Figure 3** is a graph comparing overall survival of patients with breast cancer to plasma PlGF levels at Day 14 of treatment with bevacizumab.

### Detailed Description

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., eds., 1994).

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### I. Definitions

The term "array" or "microarray", as used herein refers to an ordered arrangement of hybridizable array elements, preferably polynucleotide probes (e.g., oligonucleotides), on a substrate. The substrate can be a solid substrate, such as a glass slide, or a semi-solid substrate, such as nitrocellulose membrane. The nucleotide sequences can be DNA, RNA, or any permutations thereof.

A "target sequence," "target nucleic acid" or "target protein," as used herein, is a polynucleotide or protein of interest, the detection of which is desired. Generally, a "template," as used herein, is a polynucleotide that contains the target nucleotide sequence. In some instances, the terms "target sequence," "template DNA," "template polynucleotide," "target nucleic acid," "target polynucleotide," and variations thereof, are used interchangeably.

"Amplification," as used herein, generally refers to the process of producing multiple copies of a desired sequence. "Multiple copies" mean at least 2 copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

Expression/amount of a gene, protein or biomarker in a first sample is high or increased as compared to expression/amount in a second sample if the expression level/amount of the gene, gene product, e.g., protein or biomarker in the first sample is greater than the expression level/amount of the gene, gene product, e.g., protein or biomarker in the second sample. In one embodiment, the increase in expression level/amount of the gene, gene product, e.g., protein or biomarker in the first sample is at least about 1.5X, 1.75X, 2X, 3X, 4X, 5X, 6X, 7X, 8X, 9X, 10X, 25X, 50X, 75X, or 100X the expression level/amount of the respective gene, gene product, e.g., protein or biomarker in the second sample.

Expression/amount of a gene, protein or biomarker in a first sample is low or decreased as compared to expression/amount in a second sample if the expression level/amount of the gene, gene product, e.g., protein or biomarker in the first sample is less than the expression level/amount of the gene, gene product, e.g., protein or biomarkers in the second sample. In one embodiment, the decrease in expression level/amount of the gene, gene product, e.g., protein or biomarker in the first sample is at least about 1.5X, 1.75X, 2X, 3X, 4X, 5X, 6X, 7X, 8X, 9X, 10X, 25X, 50X, 75X, or 100X lower than the expression level/amount of the respective gene, gene product, e.g., protein or biomarker in the second sample.

Expression levels/amount can be determined based on any suitable criterion known in the art, including but not limited to mRNA, cDNA, proteins, protein fragments and/or gene copy. Expression levels/amounts can be determined qualitatively and/or quantitatively. In one embodiment, the samples are normalized for both differences in the amount of RNA or protein assayed and variability in the quality of the RNA or protein samples used. Such normalization may be accomplished by measuring and incorporating the expression of certain normalizing genes, including well known housekeeping genes, such as GAPDH. Alternatively, normalization can be based on the mean or median signal of all of the assayed genes or a large subset thereof (global normalization approach). On a gene-by-gene basis, measured normalized amount of a patient tumor mRNA or protein is compared to the amount found in a reference set. Normalized expression levels for each mRNA or protein per tested tumor per patient can be expressed as a percentage of the expression level measured in the reference set. The expression level measured in a particular patient sample to be analyzed will fall at some percentile within this range, which can be determined by methods well known in the art.

"Detection" includes any means of detecting, including direct and indirect detection.

The term "sample," or "test sample" as used herein, refers to a composition that is obtained or derived from a subject of interest that contains a cellular and/or other molecular entity that is to be characterized and/or identified, for example based on physical, biochemical, chemical and/or physiological characteristics. In one embodiment, the definition encompasses blood and other liquid samples of biological origin and tissue samples such as a biopsy specimen or tissue cultures or cells derived therefrom. The source of the tissue sample may be solid tissue as from a fresh, frozen and/or preserved organ or tissue sample or biopsy or aspirate; blood or any blood constituents; bodily fluids; and cells from any time in gestation or development of the subject or plasma. Samples may be obtained from a subject prior to commencement of treatment (e.g., cancer treatment) or after commencement of treatment (e.g., cancer treatment). Samples may be obtained within 24 hours, 7, 10, 14, 28,42, or 56 days after commencement of treatment (e.g., cancer treatment).

The term "sample," or "test sample" includes biological samples that have been manipulated in anyway after their procurement, such as by treatment with reagents, solubilization, or enrichment for certain components, such as proteins or polynucleotides, or embedding in a semi-solid or solid matrix for sectioning purposes. For the purposes herein a "section" of a tissue sample is meant a single part or piece of a tissue sample, *e.g.* a thin slice of tissue or cells cut from a tissue sample.

Samples include, but not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

In one embodiment, the sample is a clinical sample. In another embodiment, the sample is used in a diagnostic assay. In some embodiments, the sample is obtained from a primary or metastatic tumor. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood.

In one embodiment, a sample is obtained from a subject or patient prior to anti-angiogenic therapy. In another embodiment, a sample is obtained from a subject or patient prior to VEGF antagonist therapy. In yet another embodiment, a sample is obtained from a subject or patient prior to anti-VEGF antibody therapy. In even another embodiment, a sample is obtained from a subject or patient following at least one treatment with VEGF antagonist therapy.

In one embodiment, a sample is obtained from a subject or patient after at least one treatment with an anti-angiogenic therapy. In yet another embodiment, a sample is obtained from a subject or patient following at least one treatment with an anti-VEGF antibody. In some embodiments, a sample is obtained from a patient before cancer has metastasized. In certain embodiments, a sample is obtained from a patient after cancer has metastasized.

A "reference sample," as used herein, refers to any sample, standard, or level that is used for comparison purposes. In one embodiment, a reference sample is obtained from a healthy and/or non-diseased part of the body (e.g., tissue or cells) of the same subject or patient. In another embodiment, a reference sample is obtained from an untreated tissue and/or cell of the body of the same subject or patient. In yet another embodiment, a reference sample is obtained from a healthy and/or non-diseased part of the body (e.g., tissues or cells) of an individual who is not the subject or patient. In even another embodiment, a reference sample is obtained from an untreated tissue and/or cell part of the body of an individual who is not the subject or patient.

In certain embodiments, a reference sample is a single sample or combined multiple samples from the same subject or patient that are obtained at one or more different time points than when the test sample is obtained. For example, a reference sample is obtained at an earlier time point from the same subject or patient than when the test sample is obtained. Such reference sample may be useful if the reference sample is obtained during initial diagnosis of cancer and the test sample is later obtained when the cancer becomes metastatic.

In certain embodiments, a reference sample includes all types of biological samples as defined above under the term "sample" that is obtained from one or more individuals who is not the subject or patient. In certain embodiments, a reference sample is obtained from one or more individuals with an angiogenic disorder (e.g., cancer) who is not the subject or patient.

In certain embodiments, a reference sample is a combined multiple samples from one or more healthy individuals who are not the subject or patient. In certain embodiments, a reference sample is a combined multiple samples from one or more individuals with a disease or disorder (e.g., an angiogenic disorder such as, for example, cancer) who are not the subject or patient. In certain embodiments, a reference sample is pooled RNA samples from normal tissues or pooled plasma or serum samples from one or more individuals who are not the subject or patient. In certain embodiments, a reference sample is pooled RNA samples from tumor tissues or pooled plasma or serum samples from one or more individuals with a disease or disorder (e.g., an angiogenic disorder such as, for example, cancer) who are not the subject or patient.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α- anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

A "primer" is generally a short single stranded polynucleotide, generally with a free 3'-OH group, that binds to a target potentially present in a sample of interest by hybridizing with a target sequence, and thereafter promotes polymerization of a polynucleotide complementary to the target.

The term "biomarker" as used herein refers generally to a molecule, including a gene, protein, carbohydrate structure, or glycolipid, the expression of which in or on a mammalian tissue or cell can be detected by standard methods (or methods disclosed herein) and is predictive, diagnostic and/or prognostic for a mammalian cell's or tissue's sensitivity to treatment regimes based on inhibition of angiogenesis e.g. an anti-angiogenesis agent such as a VEGF-specific inhibitor. Optionally, the expression of such a biomarker is determined to be higher than that observed for a control/reference tissue or cell sample. Expression of such biomarkers can be determined using a high-throughput multiplexed immunoassay such as those commercially available from Rules Based Medicine, Inc. or Meso Scale Discovery. Expression of the biomarkers may also be determined using, e.g., PCR or FACS assay, an immunohistochemical assay or a gene chip-based assay.

By "tissue or cell sample" is meant a collection of cells obtained from a tissue of a subject or patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject or plasma. The tissue sample may also be primary or cultured cells or cell lines. Optionally, the tissue or cell sample is obtained from a cancerous tissue/organ. The tissue sample may contain compounds which are not naturally intermixed with the tissue in nature such as preservatives, anticoagulants, buffers, fixatives, nutrients, antibiotics, or the like. For the purposes herein a "section" of a tissue sample is meant a single part or piece of a tissue sample, *e.g.* a thin slice of tissue or cells cut from a tissue sample.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of gene expression analysis or protocol, one may use the results of the gene expression analysis or protocol to determine whether a specific therapeutic regimen should be performed.

The word "label" when used herein refers to a compound or composition which is conjugated or fused directly or indirectly to a reagent such as a nucleic acid probe or an antibody and facilitates detection of the reagent to which it is conjugated or fused. The label may itself be detectable *(e.g.,* radioisotope labels or fluorescent labels) or, in the case of an enzymatic label, may catalyze chemical alteration of a substrate compound or composition which is detectable.

A "native sequence" polypeptide comprises a polypeptide having the same amino acid sequence as a polypeptide derived from nature. Thus, a native sequence polypeptide can have the amino acid sequence of naturally-occurring polypeptide from any mammal. Such native sequence polypeptide can be isolated from nature or can be produced by recombinant or synthetic means. The term "native sequence" polypeptide specifically encompasses naturally-occurring truncated or secreted forms of the polypeptide (e.g., an extracellular domain sequence), naturally-occurring variant forms (e.g., alternatively spliced forms) and naturally-occurring allelic variants of the polypeptide.

A polypeptide "variant" means a biologically active polypeptide having at least about 80% amino acid sequence identity with the native sequence polypeptide. Such variants include, for instance, polypeptides wherein one or more amino acid residues are added, or deleted, at the N- or C-terminus of the polypeptide. Ordinarily, a variant will have at least about 80% amino acid sequence identity, more preferably at least about 90% amino acid sequence identity, and even more preferably at least about 95% amino acid sequence identity with the native sequence polypeptide.

The term "VEGF' or "VEGF-A" is used to refer to the 165-amino acid human vascular endothelial cell growth factor and related 121-, 189-, and 206- amino acid human vascular endothelial cell growth factors, as described by Leung et al. Science, 246:1306 (1989), and Houck et al. Mol. Endocrin., 5:1806 (1991), together with the naturally occurring allelic and processed forms thereof. VEGF-A is part of a gene family including VEGF-B, VEGF-C, VEGF-D, VEGF-E, VEGF-F, and PlGF. VEGF-A primarily binds to two high affinity receptor tyrosine kinases, VEGFR-1 (Flt-1) and VEGFR-2 (Flk-1/KDR), the latter being the major transmitter of vascular endothelial cell mitogenic signals of VEGF-A. Additionally, neuropilin-1 has been identified as a receptor for heparin-binding VEGF-A isoforms, and may play a role in vascular development. The term "VEGF' or "VEGF-A" also refers to VEGFs from non-human species such as mouse, rat, or primate. Sometimes the VEGF from a specific species is indicated by terms such as hVEGF for human VEGF or mVEGF for murine VEGF. The term "VEGF' is also used to refer to truncated forms or fragments of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "VEGF₁₆₅." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

The term "VEGF" or "VEGF-A" as used herein refers to the 165-amino acid human vascular endothelial cell growth factor and related 121-, 189-, and 206- amino acid human vascular endothelial cell growth factors, as described by Leung et aL (1989) Science 246:1306, and Houck et aL (1991) Mol. Endocrin, 5:1806, together with the naturally occurring allelic and processed forms thereof. The term "VEGF' also refers to VEGFs from non-human species such as mouse, rat or primate. Sometimes the VEGF from a specific species are indicated by terms such as hVEGF for human VEGF, mVEGF for murine VEGF, and etc. The term "VEGF" is also used to refer to truncated forms of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "VEGF₁₆₅." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

"VEGF biological activity" includes binding to any VEGF receptor or any VEGF signaling activity such as regulation of both normal and abnormal angiogenesis and vasculogenesis (Ferrara and Davis-Smyth (1997) Endocrine Rev. 18:4-25; Ferrara (1999) J. Mol. Med. 77:527-543); promoting embryonic vasculogenesis and angiogenesis (Carmeliet et al. (1996) Nature 380:435-439; Ferrara et al. (1996) Nature 380:439-442); and modulating the cyclical blood vessel proliferation in the female reproductive tract and for bone growth and cartilage formation (Ferrara et al. (1998) Nature Med. 4:336-340; Gerber et al. (1999) Nature Med. 5:623-628). In addition to being an angiogenic factor in angiogenesis and vasculogenesis, VEGF, as a pleiotropic growth factor, exhibits multiple biological effects in other physiological processes, such as endothelial cell survival, vessel permeability and vasodilation, monocyte chemotaxis and calcium influx (Ferrara and Davis-Smyth (1997), *supra* and Cebe-Suarez et al. Cell. Mol. Life Sci. 63:601-615 (2006)). Moreover, recent studies have reported mitogenic effects of VEGF on a few non-endothelial cell types, such as retinal pigment epithelial cells, pancreatic duct cells, and Schwann cells. Guerrin et al. (1995) J. Cell Physiol. 164:385-394; Oberg-Welsh et al. (1997) Mol. Cell. Endocrinol. 126:125-132; Sondell et al. (1999) J. Neurosci. 19:5731-5740.

A "VEGF antagonist" or "VEGF-specific antagonist" refers to a molecule capable of binding to VEGF, reducing VEGF expression levels, or neutralizing, blocking, inhibiting, abrogating, reducing, or interfering with VEGF biological activities, including, but not limited to, VEGF binding to one or more VEGF receptors and VEGF mediated angiogenesis and endothelial cell survival or proliferation. Included as VEGF-specific antagonists useful in the methods of the invention are polypeptides that specifically bind to VEGF, anti-VEGF antibodies and antigen-binding fragments thereof, receptor molecules and derivatives which bind specifically to VEGF thereby sequestering its binding to one or more receptors, fusions proteins (e.g., VEGF-Trap (Regeneron)), and VEGF₁₂₁-gelonin (Peregrine). VEGF-specific antagonists also include antagonist variants of VEGF polypeptides, antisense nucleobase oligomers complementary to at least a fragment of a nucleic acid molecule encoding a VEGF polypeptide; small RNAs complementary to at least a fragment of a nucleic acid molecule encoding a VEGF polypeptide; ribozymes that target VEGF; peptibodies to VEGF; and VEGF aptamers. VEGF-specific antagonists also include nonpeptide small molecules that bind to VEGF and are capable of blocking, inhibiting, abrogating, reducing, or interfering with VEGF biological activities. Thus, the term "VEGF activities" specifically includes VEGF mediated biological activities of VEGF. In certain embodiments, the VEGF antagonist reduces or inhibits, by at least 10%, 20%, 30%, 40%, 50%, 60%, 70%, 80%, 90% or more, the expression level or biological activity of VEGF. In some embodiments, the VEGF inhibited by the VEGF-specific antagonist is VEGF (8-109), VEGF (1-109), or VEGF₁₆₅.

An "anti-VEGF antibody" is an antibody that binds to VEGF with sufficient affinity and specificity. In certain embodiments, the antibody selected will normally have a sufficiently binding affinity for VEGF, for example, the antibody may bind hVEGF with a K_{d} value of between 100 nM-1 pM. Antibody affinities may be determined by a surface plasmon resonance based assay (such as the BIAcore assay as described in PCT Publication No. WO2005/012359); enzyme-linked immunoabsorbent assay (ELISA); and competition assays (e.g. RIA's), for example.

In certain embodiment, the anti-VEGF antibody can be used as a therapeutic agent in targeting and interfering with diseases or conditions wherein the VEGF activity is involved. Also, the antibody may be subjected to other biological activity assays, e.g., in order to evaluate its effectiveness as a therapeutic. Such assays are known in the art and depend on the target antigen and intended use for the antibody. Examples include the HUVEC inhibition assay; tumor cell growth inhibition assays (as described in WO 89/06692, for example); antibody-dependent cellular cytotoxicity (ADCC) and complement-mediated cytotoxicity (CDC) assays (US Patent 5,500,362); and agonistic activity or hematopoiesis assays (see WO 95/27062). An anti-VEGF antibody will usually not bind to other VEGF homologues such as VEGF-B or VEGF-C, nor other growth factors such as PlGF, PDGF or bFGF. In one embodiment, anti-VEGF antibody is a monoclonal antibody that binds to the same epitope as the monoclonal anti-VEGF antibody A4.6.1 produced by hybridoma ATCC HB 10709. In another embodiment, the anti-VEGF antibody is a recombinant humanized anti-VEGF monoclonal antibody generated according to Presta et al. (1997) Cancer Res. 57:4593-4599, including but not limited to the antibody known as bevacizumab (BV; AVASTIN^{®}).

The anti-VEGF antibody "Bevacizumab (BV)," also known as "rhuMAb VEGF" or "AVASTIN^{®}," is a recombinant humanized anti-VEGF monoclonal antibody generated according to Presta et al. (1997) Cancer Res. 57:4593-4599. It comprises mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of Bevacizumab, including most of the framework regions, is derived from human IgG1, and about 7% of the sequence is derived from the murine antibody A4.6.1. Bevacizumab has a molecular mass of about 149,000 daltons and is glycosylated. Bevacizumab and other humanized anti-VEGF antibodies are further described in U.S. Pat. No. 6,884,879 issued Feb. 26, 2005. Additional preferred antibodies include the G6 or B20 series antibodies (e.g., G6-31, B20-4.1), as described in PCT Publication No. WO2005/012359. For additional preferred antibodies see U.S. Pat. Nos. 7,060,269,6,582,959,6,703,020; 6,054,297; WO98/45332; WO 96/30046; WO94/10202; EP 0666868B1; U.S. Patent Publication Nos. 2006009360, 20050186208, 20030206899, 20030190317, 20030203409, and 20050112126; and Popkov et al., Journal of Immunological Methods 288:149-164 (2004). Other preferred antibodies include those that bind to a functional epitope on human VEGF comprising of residues F17, M18, D19, Y21, Y25, Q89, I91, K101, E103, and C104 or, alternatively, comprising residues F17, Y21, Q22, Y25, D63, I83 and Q89.

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

A "blocking" antibody or an antibody "antagonist" is one which inhibits or reduces biological activity of the antigen it binds. For example, a VEGF-specific antagonist antibody binds VEGF and inhibits the ability of VEGF to induce vascular endothelial cell proliferation. Preferred blocking antibodies or antagonist antibodies completely inhibit the biological activity of the antigen.

Unless indicated otherwise, the expression "multivalent antibody" is used throughout this specification to denote an antibody comprising three or more antigen binding sites. The multivalent antibody is preferably engineered to have the three or more antigen binding sites and is generally not a native sequence IgM or IgA antibody.

An "Fv" fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the V_{H}-V_{L} dimer. Collectively, the six CDRs or a subset thereof confer antigen binding specificity to the antibody. However, even a single variable domain (or half of an Fv comprising only three CDRs specific for an antigen) has the ability to recognize and bind antigen, although usually at a lower affinity than the entire binding site.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, *e.g.,* U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. Proc. Natl. Acad. Sci. 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Biotechnology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., J. Immunol., 147 (1):86-95 (1991); and U.S. Pat. No. 5,750,373.

An "isolated" polypeptide or "isolated" antibody is one that has been identified and separated and/or recovered from a component of its natural environment. Contaminant components of its natural environment are materials that would interfere with diagnostic or therapeutic uses for the polypeptide or antibody, and may include enzymes, hormones, and other proteinaceous or nonproteinaceous solutes. In preferred embodiments, the polypeptide or antibody will be purified (1) to greater than 95% by weight of polypeptide or antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated polypeptide or antibody includes the polypeptide or antibody in situ within recombinant cells since at least one component of the polypeptide's natural environment will not be present. Ordinarily, however, isolated polypeptide or antibody will be prepared by at least one purification step.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis. In some embodiments, methods and compositions of the invention are useful in attempts to delay development of a disease or disorder.

An "effective amount" refers to an amount effective, at dosages and for periods of time necessary, to achieve the desired therapeutic or prophylactic result. A "therapeutically effective amount" of a therapeutic agent may vary according to factors such as the disease state, age, sex, and weight of the individual, and the ability of the antibody to elicit a desired response in the individual. A therapeutically effective amount is also one in which any toxic or detrimental effects of the therapeutic agent are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the case of pre-cancerous, benign, early or late-stage tumors, the therapeutically effective amount of the angiogenic inhibitor may reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, and/or quality of life.

"Short progression free survival" refers to progression at the time of first tumor assessment. Depending on the type of cancer or tumor the first time of tumor assessment occurs about 4, 3, 2 or 1 month after initiation of treatment. Timing of first tumor assessment depends on how fast the particular disease progresses. In one embodiment the time of first tumor assessment for renal cancer is 56 days after commencement of anti-cancer therapy.

The terms "cancer" and "cancerous" refer to or describe the physiological condition in mammals that is typically characterized by unregulated cell growth. Included in this definition are benign and malignant cancers. Examples of cancer include but are not limited to, carcinoma, lymphoma, blastoma, sarcoma, and leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

By "subject" or "patient" is meant a mammal, including, but not limited to, a human or non-human mammal, such as a bovine, equine, canine, ovine or feline. Preferably, the subject or patient is a human.

The term "anti-cancer therapy" refers to a therapy useful in treating cancer. Examples of anti-cancer therapeutic agents include, but are limited to, e.g., chemotherapeutic agents, growth inhibitory agents, cytotoxic agents, agents used in radiation therapy, anti-angiogenesis agents, apoptotic agents, anti-tubulin agents, and other agents to treat cancer, such as anti-HER-2 antibodies, anti-CD20 antibodies, an epidermal growth factor receptor (EGFR) antagonist (e.g., a tyrosine kinase inhibitor), HER1/EGFR inhibitor (e.g., erlodnib (Tarceva^{™}), platelet derived growth factor inhibitors (e.g., Gleevec^{™} (Imatinib Mesylate)), a COX-2 inhibitor (e.g., celecoxib), interferons, cytokines, antagonists (e.g., neutralizing antibodies) that bind to one or more of the following targets VEGF, ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also included in the invention.

An "angiogenic factor or agent" is a growth factor or its receptor which is involved in stimulating the development of blood vessels, *e.g.,* promote angiogenesis, endothelial cell growth, stabiliy of blood vessels, and/or vasculogenesis, *etc.* For example, angiogenic factors, include, but are not limited to, *e.g.,* VEGF and members of the VEGF family and their receptors (VEGF-B, VEGF-C, VEGF-D, VEGFR1, VEGFR2 and VEGFR3), PlGF, PDGF family, fibroblast growth factor family (FGFs), TIE ligands (Angiopoietins, ANGPT1, ANGPT2), TIE1, TIE2, ephrins, Bv8, Delta-like ligand 4 (DLL4), Del-1, fibroblast growth factors: acidic (aFGF) and basic (bFGF), FGF4, FGF9, BMP9, BMP10, Follistatin, Granulocyte colony-stimulating factor (G-CSF), GM-CSF, Hepatocyte growth factor (HGF) /scatter factor (SF), Interleukin-8 (IL-8), CXCL12, Leptin, Midkine, neuropilins, NRP1, NRP2, Placental growth factor, Platelet-derived endothelial cell growth factor (PD-ECGF), Platelet-derived growth factor, especially PDGF-BB, PDGFR-alpha, or PDGFR-beta, Pleiotrophin (PTN), Progranulin, Proliferin, Transforming growth factor-alpha (TGF-alpha), Transforming growth factor-beta (TGF-beta), Tumor necrosis factor-alpha (TNF-alpha), Alkl, CXCR4, Notch1, Notch4, Sema3A, Sema3C, Sema3F, Robo4, *etc.* It would further include factors that promote angiogenesis, such as ESM1 and Perlecan. It would also include factors that accelerate wound healing, such as growth hormone, insulin-like growth factor-I (IGF-I), VIGF, epidermal growth factor (EGF), EGF-like domain, multiple 7 (EGFL7), CTGF and members of its family, and TGF-alpha and TGF-beta. *See, e.g.,* Klagsbrun and D'Amore (1991) Annu. Rev. Physiol. 53:217-39; Streit and Detmar (2003) Oncogene 22:3172-3179; Ferrara & Alitalo (1999) Nature Medicine 5(12):1359-1364; Tonini et al. (2003) Oncogene 22:6549-6556 (e.g., Table 1 listing known angiogenic factors); and, Sato (2003) Int. J. Clin. Oncol. 8:200-206.

An "anti-angiogenic agent," "angiogenic inhibitor," "anti-angiogenesis agent," or "angiogenesis inhibitor" refers to a small molecular weight substance, a polynucleotide (including, e.g., an inhibitory RNA (RNAi or siRNA)), a polypeptide, an isolated protein, a recombinant protein, an antibody, or conjugates or fusion proteins thereof, that inhibits angiogenesis, vasculogenesis, or undesirable vascular permeability, either directly or indirectly. It should be understood that the anti-angiogenic agent includes those agents that bind and block the angiogenic activity of the angiogenic factor or its receptor. For example, an anti-angiogenic agent is an antibody or other antagonist to an angiogenic agent as defined above, e.g., antibodies to VEGF-A or to the VEGF-A receptor (e.g., KDR receptor or Flt-1 receptor), anti-PDGFR inhibitors (e.g., Gleevec® (imatinib mesylate)), small molecules that block VEGF receptor signaling (e.g., PTK787/ZK2284, SU6668, SUTENT®/SU11248 (sunitinib malate), AMG706, or those described in, e.g., international patent publication WO 2004/113304). Anti-angiogenic agents include, but are not limited to, the following agents: VEGF inhibitors such as a VEGF-specific antagonist, EGF inhibitor, EGFR inhibitors, Erbitux® (cetuximab, ImClone Systems, Inc., Branchburg, N.J.), Vectibix® (panitumumab, Amgen, Thousand Oaks, CA), TIE2 inhibitors, IGF1R inhibitors, COX-II (cyclooxygenase II) inhibitors, MMP-2 (matrix-metalloproteinase 2) inhibitors, and MMP-9 (matrix-metalloproteinase 9) inhibitors, CP-547,632 (Pfizer Inc., NY, USA), Axitinib (Pfizer Inc.; AG-013736), ZD-6474 (AstraZeneca), AEE788 (Novartis), AZD-2171), VEGF Trap (Regeneron/Aventis), Vatalanib (also known as PTK-787, ZK-222584: Novartis & Schering A G), Macugen (pegaptanib octasodium, NX-1838, EYE-001, Pfizer Inc./Gilead/Eyetech), IM862 (Cytran Inc. of Kirkland, Wash., USA); and angiozyme, a synthetic ribozyme from Ribozyme (Boulder, Colo.) and Chiron (Emeryville, Calif.) and combinations thereof. Other angiogenesis inhibitors include thrombospondin1, thrombospondin2, collagen IV and collagen XVIII. VEGF, inhibitors are disclosed in U.S. Pat. Nos. 6,534,524 and 6,235,764. Anti-angiogenic agents also include native angiogenesis inhibitors , e.g., angiostatin, endostatin, etc. *See, e.g.,* Klagsbrun and D'Amore (1991) Annu. Rev. Physiol. 53:217-39; Streit and Detmar (2003) Oncogene 22:3172-3179 (e.g., Table 3 listing anti-angiogenic therapy in malignant melanoma); Ferrara & Alitalo (1999) Nature Medicine 5(12):1359-1364; Tonini et al. (2003) Oncogene 22:6549-6556 (e.g., Table 2 listing known antiangiogenic factors); and, Sato (2003) Int. J. Clin. Oncol. 8:200-206 (e.g., Table 1 listing anti-angiogenic agents used in clinical trials).

The term "anti-angiogenic therapy" refers to a therapy useful for inhibiting angiogenesis which comprises the administration of an anti-angiogenic agent.

The term "cytotoxic agent" as used herein refers to a substance that inhibits or prevents the function of cells and/or causes destruction of cells. The term is intended to include radioactive isotopes (*e.g*., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol- Myers Squibb Oncology, Princeton, N.J.), ABRAXANE™ Cremophor-free, albumin-engineered nanoparticle formulation of paclitaxel (American Pharmaceutical Partners, Schaumberg, Illinois), and TAXOTERE® doxetaxel (Rhône- Poulenc Rorer, Antony, France); chloranbucil; GEMZAR® gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluorometlhylornithine (DMFO); retinoids such as retinoic acid; capecitabine; combretastatin; leucovorin (LV); oxaliplatin, including the oxaliplatin treatment regimen (FOLFOX); inhibitors of PKC-alpha, Raf, H-Ras, EGFR (e.g, erlotinib (Tarceva^{™})) and VEGF-A that reduce cell proliferation and pharmaceutically acceptable salts, acids or derivatives of any of the above.

Also included in this definition are anti-hormonal agents that act to regulate or inhibit hormone action on tumors such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON- toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Raf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; Vinorelbine and Esperamicins (see U.S. Pat. No. 4,675,187), and pharmaceutically acceptable salts, acids or derivatives of any of the above.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

To "reduce or inhibit" is to decrease or reduce an activity, function, and/or amount as compared to a reference. By "reduce or inhibit" is meant the ability o cause an overall decrease preferably of 20% or greater, more preferably of 50% or greater, and most preferably of 75%, 85%, 90%, 95%, or greater. Reduce or inhibit can refer to the symptoms of the disorder being treated, the presence or size of metastases, the size of the primary tumor, or the size or number of the blood vessels in angiogenic disorders.

The term "diagnosis" is used herein to refer to the identification of a molecular or pathological state, disease or condition, such as the identification of cancer or to refer to identification of a cancer patient who may benefit from a particular treatment regimen. The term "prognosis" is used herein to refer to the prediction of the likelihood of clinical benefit from anti-cancer therapy. The term "prediction" is used herein to refer to the likelihood that a patient will respond either favorably or unfavorably to a particular anti-cancer therapy. In one embodiment, the prediction relates to the extent of those responses. In one embodiment, the prediction relates to whether and/or the probability that a patient will survive or improve following treatment, for example treatment with a particular therapeutic agent, and for a certain period of time without disease recurrence. The predictive methods of the invention can be used clinically to make treatment decisions by choosing the most appropriate treatment modalities for any particular patient. The predictive methods of the present invention are valuable tools in predicting if a patient is likely to respond favorably to a treatment regimen, such as a given therapeutic regimen, including for example, administration of a given therapeutic agent or combination, surgical intervention, steroid treatment, etc., or whether long-term survival of the patient, following a therapeutic regimen is likely.

"Patient response" can be assessed using any endpoint indicating a benefit to the patient, including, without limitation, (1) inhibition, to some extent, of disease progression, including slowing down and complete arrest; (2) reduction in lesion size; (3) inhibition (i.e., reduction, slowing down or complete stopping) of disease cell infiltration into adjacent peripheral organs and/or tissues; (4) inhibition (i.e. reduction, slowing down or complete stopping) of disease spread; (5) relief, to some extent, of one or more symptoms associated with the disorder; (6) increase in the length of disease-free presentation following treatment; and/or (8) decreased mortality at a given point of time following treatment.

The term "long-term survival" is used herein to refer to survival for at least 1 year, 5 years, 8 years, or 10 years following therapeutic treatment.

The term "benefit" is used in the broadest sense and refers to any desirable effect and specifically includes clinical benefit as defined herein.

Clinical benefit can be measured by assessing various endpoints, *e.g.,* inhibition, to some extent, of disease progression, including slowing down and complete arrest; reduction in the number of disease episodes and/or symptoms; reduction in lesion size; inhibition (*i.e.,* reduction, slowing down or complete stopping) of disease cell infiltration into adjacent peripheral organs and/or tissues; inhibition (i.e. reduction, slowing down or complete stopping) of disease spread; decrease of auto-immune response, which may, but does not have to, result in the regression or ablation of the disease lesion; relief, to some extent, of one or more symptoms associated with the disorder; increase in the length of disease-free presentation following treatment, e.g., progression-free survival; increased overall survival; higher response rate; and/or decreased mortality at a given point of time following treatment.

### II. Methods of the Invention

The present invention is based partly on the identification of specific biomarkers that correlate with reduced clinical benefit of anti-angiogenic therapy for treating cancer. Thus, the disclosed methods and assays provide convenient, efficient, and potentially cost-effective means to obtain data and information useful in assessing appropriate or effective therapies for treating cancer patients. For example, a cancer patient could have a biopsy performed to obtain a tissue or cell sample, or a plasma sample could be obtained from the patient, and the sample could be examined by various *in vitro* assays to determine whether the expression of PlGF is increased as compared to a control or reference sample. If an increase in expression is detected the patient will probably benefit from anti-cancer therapy other than or in addition to anti-angiogenic therapy. Thus, the invention provides a method of identifying a cancer patient who may benefit from anti-cancer therapy other than or in addition to anti-angiogenic therapy, comprising the step of detecting the expression level of placental growth factor (PlGF) in a sample obtained from the patient wherein increased expression of PlGF in the sample as compared to a reference sample indicates that the patient may benefit from anti-cancer therapy other than or in addition to anti-angiogenic therapy. In some embodiments the sample is obtained from the patient prior to commencement of cancer treatment, where the sample has a PlGF expression level of greater than 28 pg/ml. In some embodiments the sample is obtained from a patient currently undergoing anti-angiogenic therapy, where the sample has a PlGF expression level of greater than 50 pg/ml. In some embodiments the samples are plasma samples.

The invention further provides a method of predicting responsiveness of cancer patient to anti-angiogenic therapy comprising determining the expression level of PlGF in a sample obtained from the patient wherein increased expression levels of PlGF as compared to a reference sample indicates that the patient is less likely to be responsive to the anti-angiogenic therapy alone. In some embodiments the sample is obtained from the patient prior to commencement of cancer treatment, where the sample has a PlGF expression level of greater than 28 pg/ml. In some embodiments the sample is obtained from a patient currently undergoing anti-angiogenic therapy, where the sample has a PlGF expression level of greater than 50 pg/ml. In some embodiments the samples are plasma samples. Also provided is a method of treating a patient with cancer comprising administering to the patient anti-cancer therapy other than or in addition to anti-angiogenic therapy wherein a sample obtained from the patient show increased expression levels of PlGF as compared to a reference sample.

In another aspect the invention provides a method of optimizing therapeutic efficacy for treatment of cancer, comprising determining the expression level of PlGF in a sample obtained from the patient wherein increased expression levels of PlGF as compared to a reference sample indicates that the patient is less likely to be responsive to the anti-angiogenic therapy alone. The sample may be obtained from the patient before commencement of treatment. The sample may be obtained from the patient after the commencement of treatment. In one embodiment the sample has a PlGF expression level of less than or equal to 28 pg/ml. In some embodiments the sample may be obtained from a patient currently being treated with anti-angiogenic therapy. In one embodiment the sample has a PlGF expression level of less than or equal to 50 pg/ml. In some embodiments, the anti-angiogenic therapy comprises administration of a VEGF antagonist such as, for example, an anti-VEGF antibody, e.g., bevacizumab.

In another aspect the invention provides a method of treating a cancer patient comprising administering to the patient an anti-angiogenic therapy, wherein a sample obtained from the patient shows low expression levels of PlGF as compared to a reference sample. The sample may be obtained from the patient before commencement of treatment. In one embodiment the sample has a PlGF expression level of less than or equal to 28 pg/ml. In some embodiments the sample may be obtained from a patient currently being treated with anti-angiogenic therapy. In one embodiment the sample has a PlGF expression level of less than or equal to 50 pg/ml. In some embodiments, the anti-angiogenic therapy comprises administration of a VEGF. antagonist such as, for example, an anti-VEGF antibody, e.g., bevacizumab.

The methods of the invention involve patients with cancer. The cancer may be, e.g., carcinoma, lymphoma, blastema, sarcoma, and/or leukemia. More particular examples of such cancers include squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors) or Meigs' syndrome. In one embodiment the cancer is renal cell carcinoma.

A sample comprising a target biomarker can be obtained by methods well known in the art, and that are appropriate for the particular type and location of the cancer of interest. Tissue biopsy is often used to obtain a representative piece of cancerous tissue. Alternatively, cells can be obtained indirectly in the form of tissues/fluids that are known or thought to contain the cancer cells of interest. For instance, samples of cancerous lesions may be obtained by resection, bronchoscopy, fine needle aspiration, bronchial brushings, or from sputum, pleural fluid or blood. Genes or gene products can be detected from cancer or tumor tissue or from other body samples such as urine, sputum, serum or plasma. The same techniques discussed above for detection of target genes or gene products in cancerous samples can be applied to other body samples. Cancer cells may be sloughed off from cancer lesions and appear in such body samples. By screening such body samples, a simple early diagnosis can be achieved for these cancers. In addition, the progress of therapy can be monitored more easily by testing such body samples for target genes or gene products.

Means for enriching a tissue preparation for cancer cells are known in the art. For example, the tissue may be isolated from paraffin or cryostat sections. Cancer cells may also be separated from normal cells by flow cytometry or laser capture microdissection. These, as well as other techniques for separating cancerous from normal cells, are well known in the art. If the cancer tissue is highly contaminated with normal cells, detection of signature gene or protein expression profile may be more difficult, although techniques for minimizing contamination and/or false positive/negative results are known, some of which are described herein below. For example, a sample may also be assessed for the presence of a biomarker known to be associated with a cancer cell of interest but not a corresponding normal cell, or vice versa.

In the methods of the invention, a mammalian tissue or cell sample is obtained and examined for expression of one or more biomarkers (e.g., PlGF). Expression of various biomarkers in a sample can be analyzed by a number of methodologies, many of which are known in the art and understood by the skilled artisan, including but not limited to, immunohistochemical and/or Western blot analysis, immunoprecipitation, molecular binding assays, ELISA, ELIFA, fluorescence activated cell sorting (FACS) and the like, quantitative blood based assays (as for example Serum ELISA) (to examine, for example, levels of protein expression), biochemical enzymatic activity assays, *in situ* hybridization, Northern analysis and/or PCR analysis of mRNAs, as well as any one of the wide variety of assays that can be performed by gene and/or tissue array analysis. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery (MSD) may also be used.

In some embodiments of the invention, the expression of target proteins in a sample is examined using immunohistochemistry and staining protocols. Immunohistochemical staining of tissue sections has been shown to be a reliable method of assessing or detecting presence of proteins in a sample. Immunohistochemistry ("IHC") techniques utilize an antibody to probe and visualize cellular antigens *in situ*, generally by chromogenic or fluorescent methods.

For sample preparation, a tissue or cell sample from a mammal (typically a human patient) may be used. Examples of samples include, but are not limited to, tissue biopsy, blood, lung aspirate, sputum, lymph fluid, plasma etc. The sample can be obtained by a variety of procedures known in the art including, but not limited to surgical excision, aspiration or biopsy. The tissue may be fresh or frozen. In one embodiment, the sample is fixed and embedded in paraffin or the like.

The tissue sample may be fixed (*i.e.* preserved) by conventional methodology (See *e.g.,* "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C.). One of skill in the art will appreciate that the choice of a fixative is determined by the purpose for which the sample is to be histologically stained or otherwise analyzed. One of skill in the art will also appreciate that the length of fixation depends upon the size of the tissue sample and the fixative used. By way of example, neutral buffered formalin, Bouin's or paraformaldehyde, may be used to fix a sample.

Generally, the sample is first fixed and is then dehydrated through an ascending series of alcohols, infiltrated and embedded with paraffin or other sectioning media so that the tissue sample may be sectioned. Alternatively, one may section the tissue and fix the sections obtained. By way of example, the tissue sample may be embedded and processed in paraffin by conventional methodology (See *e.g.,* "Manual of Histological Staining Method of the Armed Forces Institute of Pathology", *supra*). Examples of paraffin that may be used include, but are not limited to, Paraplast, Broloid, and Tissuemay. Once the tissue sample is embedded, the sample may be sectioned by a microtome or the like (See *e.g.,* "Manual of Histological Staining Method of the Armed Forces Institute of Pathology", *supra*). By way of example for this procedure, sections may range from about three microns to about five microns in thickness. Once sectioned, the sections may be attached to slides by several standard methods. Examples of slide adhesives include, but are not limited to, silane, gelatin, poly-L-lysine and the like. By way of example, the paraffin embedded sections may be attached to positively charged slides and/or slides coated with poly-L-lysine.

If paraffin has been used as the embedding material, the tissue sections are generally deparaffinized and rehydrated to water. The tissue sections may be deparaffinized by several conventional standard methodologies. For example, xylenes and a gradually descending series of alcohols may be used (See *e.g*., "Manual of Histological Staining Method of the Armed Forces Institute of Pathology", *supra*). Alternatively, commercially available deparaffinizing non-organic agents such as Hemo-De7 (CMS, Houston, Texas) may be used.

Optionally, subsequent to the sample preparation, a tissue section may be analyzed using IHC. IHC may be performed in combination with additional techniques such as morphological staining and/or fluorescence *in-situ* hybridization. Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for immunohistochemistry typically will be labeled with a detectable moiety. Numerous labels are available which can be generally grouped into the following categories:
(a) Radioisotopes, such as ³⁵S, ¹⁴C, ¹²⁵I, ³H, and ¹³¹I. The antibody can be labeled with the radioisotope using the techniques described in Current Protocols in Immunology, Volumes 1 and 2, Coligen et al., Ed. Wiley-Interscience, New York, New York, Pubs. (1991) for example and radioactivity can be measured using scintillation counting.
(b) Colloidal gold particles.
(c) Fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above. The fluorescent labels can be conjugated to the antibody using the techniques disclosed in *Current Protocols in Immunology, supra,* for examples. Fluorescence can be quantified using a fluorimeter.
(d) Various enzyme-substrate labels are available and U.S. Patent No. 4,275,149 provides a review of some of these. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases *(e.g.,* firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase,

β-galactosidase, glucoamylase, lysozyme, saccharide oxidases (*e.g*., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed. J. Langone & H. Van Vunakis), Academic press, New York, 73:147-166 (1981).

Examples of enzyme-substrate combinations include, for example:
(i) Horseradish peroxidase (HRPO) with hydrogen peroxidase as a substrate, wherein the hydrogen peroxidase oxidizes a dye precursor (*e.g.,* orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (*e.g.,* p-nitrophenyl-β-D-galactosidase) or fluorogenic substrate (*e.g*., 4-methylumbelliferyl-β-D-galactosidase).

Numerous other enzyme-substrate combinations are available to those skilled in the art. For a general review of these, see U.S. Patent Nos. 4,275,149 and 4,318,980. Sometimes, the label is indirectly conjugated with the antibody. The skilled artisan will be aware of various techniques for achieving this. For example, the antibody can be conjugated with biotin and any of the four broad categories of labels mentioned above can be conjugated with avidin, or *vice versa.* Biotin binds selectively to avidin and thus, the label can be conjugated with the antibody in this indirect manner. Alternatively, to achieve indirect conjugation of the label with the antibody, the antibody is conjugated with a small hapten and one of the different types of labels mentioned above is conjugated with an anti-hapten antibody. Thus, indirect conjugation of the label with the antibody can be achieved.

Aside from the sample preparation procedures discussed above, further treatment of the tissue section prior to, during or following IHC may be desired. For example, epitope retrieval methods, such as heating the tissue sample in citrate buffer may be carried out (see, *e.g.,* Leong et al. Appl. Immunohistochem. 4(3):201 (1996)).

Following an optional blocking step, the tissue section is exposed to primary antibody for a sufficient period of time and under suitable conditions such that the primary antibody binds to the target protein antigen in the tissue sample. Appropriate conditions for achieving this can be determined by routine experimentation. The extent of binding of antibody to the sample is determined by using any one of the detectable labels discussed above. Preferably, the label is an enzymatic label (*e.g*. HRPO) which catalyzes a chemical alteration of the chromogenic substrate such as 3,3'-diaminobenzidine chromogen. Preferably the enzymatic label is conjugated to antibody which binds specifically to the primary antibody (*e.g.* the primary antibody is rabbit polyclonal antibody and secondary antibody is goat anti-rabbit antibody). Specimens thus prepared may be mounted and coverslipped. Slide evaluation is then determined, *e.g.* using a microscope, and staining intensity criteria, routinely used in the art, may be employed.

In alternative methods, the sample may be contacted with an antibody specific for said biomarker under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labelled antibody to a target biomarker.

Sandwich assays are among the most useful and commonly used assays. A number of variations of the sandwich assay technique exist, and all are intended to be encompassed by the present invention. Briefly, in a typical forward assay, an unlabelled antibody is immobilized on a solid substrate, and the sample to be tested brought into contact with the bound molecule. After a suitable period of incubation, for a period of time sufficient to allow formation of an antibody-antigen complex, a second antibody specific to the antigen, labelled with a reporter molecule capable of producing a detectable signal is then added and incubated, allowing time sufficient for the formation of another complex of antibody-antigen-labelled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labelled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40°C such as between 25° C and 32° C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labelled with a reporter molecule. Depending on the amount of target and the strength of the reporter molecule signal, a bound target may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, -galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labelled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic color visually detectable with a light microscope. As in the EIA, the fluorescent labelled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

It is contemplated that the above described techniques may also be employed to detect expression of PlGF.

Methods of the invention further include protocols which examine the presence and/or expression of PlGF mRNA levels in a tissue, cell or plasma sample. Methods for the evaluation of mRNAs in cells are well known and include, for example, hybridization assays using complementary DNA probes (such as *in situ* hybridization using labeled riboprobes specific for PlGF, Northern blot and related techniques) and various nucleic acid amplification assays (such as RT-PCR using complementary primers specific for PlGF, and other amplification type detection methods, such as, for example, branched DNA, SISBA, TMA and the like).

Tissue, cell or plasma samples from mammals can be conveniently assayed for mRNAs using Northern, dot blot or PCR analysis. For example, RT-PCR assays such as quantitative PCR assays are well known in the art. In an illustrative embodiment of the invention, a method for detecting a target mRNA in a biological sample comprises producing cDNA from the sample by reverse transcription using at least one primer; amplifying the cDNA so produced using a target polynucleotide as sense and antisense primers to amplify target cDNAs therein; and detecting the presence of the amplified target cDNA. In addition, such methods can include one or more steps that allow one to determine the levels of target mRNA in a biological sample (e.g. by simultaneously examining the levels a comparative control mRNA sequence of a "housekeeping" gene such as an actin family member). Optionally, the sequence of the amplified target cDNA can be determined.

Optional methods of the invention include protocols which examine or detect mRNAs, such as target mRNAs, in a tissue, cell or plasma sample by microarray technologies. Using nucleic acid microarrays, test and control mRNA samples from test and control samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlate with increased or reduced clinical benefit of anti-angiogenic therapy may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment. (see, e.g., WO 01/75166 published October 11, 2001; (See, for example, U.S. 5,700,637, U.S. Patent 5,445,934, and U.S. Patent 5,807,522, Lockart, Nature Biotechnology, 14:1675-1680 (1996); Cheung, V.G. et al., Nature Genetics 21(Suppl):15-19 (1999) for a discussion of array fabrication), DNA microarrays are miniature arrays containing gene fragments that are either synthesized directly onto or spotted onto glass or other substrates. Thousands of genes are usually represented in a single array. A typical microarray experiment involves the following steps: 1) preparation of fluorescently labeled target from RNA isolated from the sample, 2) hybridization of the labeled target to the microarray, 3) washing, staining, and scanning of the array, 4) analysis of the scanned image and 5) generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (in situ).

The Affymetrix GeneChip® system is a commerically available microarray system which comprises arrays fabricated by direct synthesis of oligonucleotides on a glass surface. Probe/Gene Arrays: Oligonucleotides, usually 25 mers, are directly synthesized onto a glass wafer by a combination of semiconductor-based photolithography and solid phase chemical synthesis technologies. Each array contains up to 400,000 different oligos and each oligo is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligo. The Microarray Suite software subtracts the hybridization intensities of the mismatch probes from those of the perfect match probes to determine the absolute or specific intensity value for each probe set. Probes are chosen based on current information from Genbank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

Expression of a selected biomarker in a tissue or cell sample may also be examined by way of functional or activity-based assays. For instance, if the biomarker is an enzyme, one may conduct assays known in the art to determine or detect the presence of the given enzymatic activity in the tissue or cell sample.

The kits of the invention have a number of embodiments. A typical embodiment is a kit comprising a container, a label on said container, and a composition contained within said container; wherein the composition includes a primary antibody that binds to PlGF, the label on the container indicating that the composition can be used to evaluate the presence of PlGF in at least one type of mammalian cell, and instructions for using the antibody for evaluating the presence of PlGF in at least one type of mammalian cell. The kit can further comprise a set of instructions and materials for preparing a tissue, cell or plasma sample and applying antibody and probe to the same section of a tissue, cell or plasma sample. The kit may include both a primary and secondary antibody, wherein the secondary antibody is conjugated to a label, *e.g.,* an enzymatic label.

Another embodiment is a kit comprising a container, a label on said container, and a composition contained within said container; wherein the composition includes one or more polynucleotides that hybridize to PlGF under stringent conditions, the label on said container indicates that the composition can be used to evaluate the presence of PlGF in at least one type of mammalian cell or plasma, and instructions for using the polynucleotide for evaluating the presence of PlGF RNA or DNA in at least one type of mammalian cell or plasma.

Other optional components in the kit include one or more buffers (*e.g*., block buffer, wash buffer, substrate buffer, etc), other reagents such as substrate (*e.g.,* chromogen) which is chemically altered by an enzymatic label, epitope retrieval solution, control samples (positive and/or negative controls), control slide(s) etc.

For the methods of the invention, the anti-cancer therapeutic agents, anti-angiogenesis agents and/or chemotherapeutic agents are administered to a human patient, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred.

The treatment of the present invention may involve the combined administration of an anti-VEGF antibody and one or more chemotherapeutic agents. The present invention contemplates administration of cocktails of different chemotherapeutic agents. The combined administration includes coadministration, using separate formulations or a single pharmaceutical formulation, and consecutive administration in either order, wherein preferably there is a time period while both (or all) active agents simultaneously exert their biological activities. Preparation and dosing schedules for such chemotherapeutic agents may be used according to manufacturers' instructions or as determined empirically by the skilled practitioner. Preparation and dosing schedules for chemotherapy are also described in Chemotherapy Service Ed., M.C. Perry, Williams & Wilkins, Baltimore, MD (1992). The chemotherapeutic agent may precede, or follow administration of the antibody or may be given simultaneously therewith.

For the prevention or treatment of disease, the appropriate dosage of the anti-cancer therapeutic agent or anti-angiogenesis agent will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments. In a combination therapy regimen, the compositions of the present invention are administered in a therapeutically effective or synergistic amount. In some embodiments, the anti-angiogenesis agent is VEGF antagonist, e.g., an anti-VEGF antibody such as bevacizumab. Depending on the type and severity of the disease, about 1 pg/kg to 50 mg/kg (*e.g.* 0.1-20mg/kg) of antibody is an initial candidate dosage for administration to the patient, whether, for example, by one or more separate administrations, or by continuous infusion. A typical daily dosage might range from about 1 µg/kg to about 100 mg/kg or more, depending on the factors mentioned above. For repeated administrations over several days or longer, depending on the condition, the treatment is sustained until a desired suppression of disease symptoms occurs. However, other dosage regimens may be useful. In a preferred aspect, the antibody of the invention is administered every two to three weeks, at a dose ranged from about 5mg/kg to about 15 mg/kg. More preferably, such dosing regimen is used in combination with a chemotherapy regimen as the first line therapy for treating metastatic colorectal cancer. In some aspects, the chemotherapy regimen involves the traditional high-dose intermittent administration. In some other aspects, the chemotherapeutic agents are administered using smaller and more frequent doses without scheduled breaks ("metronomic chemotherapy"). The progress of the therapy of the invention is easily monitored by conventional techniques and assays.

In some embodiments the anti-VEGF antibody used in the methods of the invention is bevacizumab. In certain embodiments, e.g., when used in combination, bevacizumab is administered in the range from about 0.05 mg/kg to about 15 mg/kg. In one embodiment, one or more doses of about 0.5 mg/kg, 1.0 mg/kg, 2.0 mg/kg, 3.0 mg/kg, 4.0 mg/kg, 5.0 mg/kg, 6.0 mg/kg, 7.0 mg/kg, 7.5 mg/kg, 8.0 mg/kg, 9.0 mg/kg, 10 mg/kg or 15 mg/kg (or any combination thereof) may be administered to the subject. Such doses may be administered intermittently, *e.g.* every day, every three days, every week or every two to three weeks. In another embodiment, e.g., when used in combination, bevacizumab is administered intravenously to the subject at 10 mg/kg every other week or 15mg/kg every three weeks.

The following examples are provided for illustrative purposes only and are not to be construed as limiting upon the teachings herein.

### EXAMPLES

### Example 1 Higher baseline PlGF levels in cancer patients correlate with shorter survival

### Study Subject and Treatment:

Samples were collected from AVF-0776, a study of bevacizumab monotherapy in patients with metastatic breast cancer who had relapsed following at least one conventional chemotherapy regimen for metastatic disease. All subjects received 3 mg/kg, 10 mg/kg or 20 mg/kg bevacizumab every 2 weeks for a total of six infusions. Subjects without evidence of disease progression at the Day 70 tumor assessment continued to receive bevacizumab every 2 weeks through Day 154 (six more infusions) and then underwent a tumor assessment. Subjects without evidence of disease progression at Day 154 received a final infusion of bevacizumab on Day 168. Subjects who experienced disease progression were discontinued from the study. Following study completion or withdrawal, subjects were followed for 1-year survival status after Day 0.

### Samples and methods:

Peripheral vein blood from subjects was drawn directly into a BD Vacutainer^{®} plastic EDTA tube with a lavender conventional stopper. After collection, samples were mixed gently and allowed to sit for 30 minutes. Plasma was obtained by centrifuging at 3,000 x g for 20 minutes in room temperature. Samples were selected from a total of 67 patients with relapsed metastatic breast cancer treated with bevacizumab and from whom plasma samples were available at least at two time points from Day 0 to Day 56 (Day 0, Day 14, Day 28, Day 42 and Day 56).

Baseline PlGF levels in plasma were measured in patients prior to commencement of treatment on Day 0 with the MSD^{®} MS6000 human growth factor kit by (K11029C-1 Meso Scale Discovery, Gaithersburg, MD) according to the manufacturer's specifications. To correlate PlGF levels with clinical outcome, the Kaplan-Meier methodology and log-rank (Mantel-Cox) test were used for the analyses of duration of survival from study initiation (first dose).

### Results:

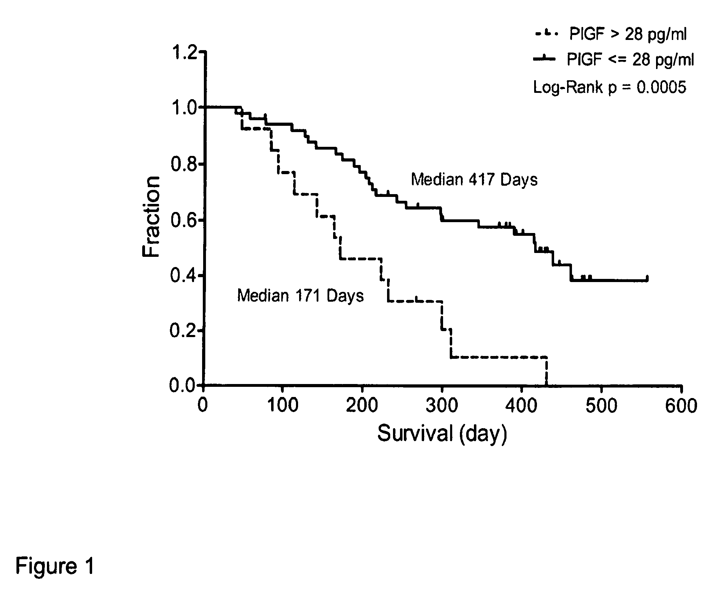

The log-rank test indentified that plasma PlGF levels at 28 pg/ml at baseline was a significant variable that correlated with overall survival of a patient ( p < 0.0026; see Figure 1). The median overall survival time was 171 days in patients with baseline plasma PlGF levels greater than 28 pg/ml at Day 0 prior to starting treatment. In contrast, the median overall survival time for patients with baseline PlGF levels less than or equal to 28 pg/ml at Day 0 prior to starting treatment was 417 days. These results indicate that baseline plasma PlGF level in a patient is a predictor of survival benefit for cancer patients undergoing anti-angiogenesis therapy.

### Example 2: Plasma PlGF levels increase in patients treated with Bevacizumab

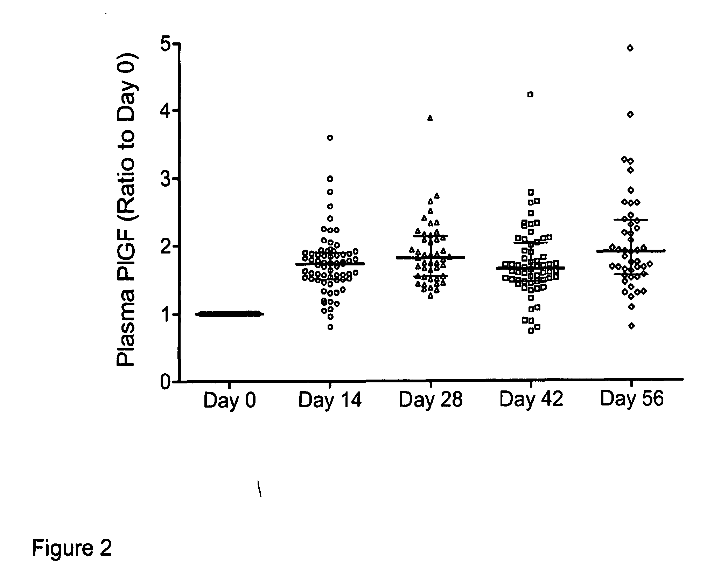

Patients were treated and plasma samples were collected as described above in Example 1. Plasma PlGF levels were monitored over the course of treatment with bevacizumab. Results show that plasma PlGF levels increased after treatment with bevacizumab (see Figure 2). Median increases of over 1.5 fold were observed in patients at Days 14,28,42, and 56 after treatment with bevacizumab.

### Example 3: Higher plasma PLGF levels in patients after treatment with bevacizumab correlate with shorter overall survival

Patients were treated, plasma samples, and plasma PlGF levels were measured as described above in Example 1. To correlate plasma PlGF levels with clinical outcome, the Kaplan-Meier methodology and log-rank (Mantel-Cox) test were used for the analyses of duration of survival from study initiation (first dose).

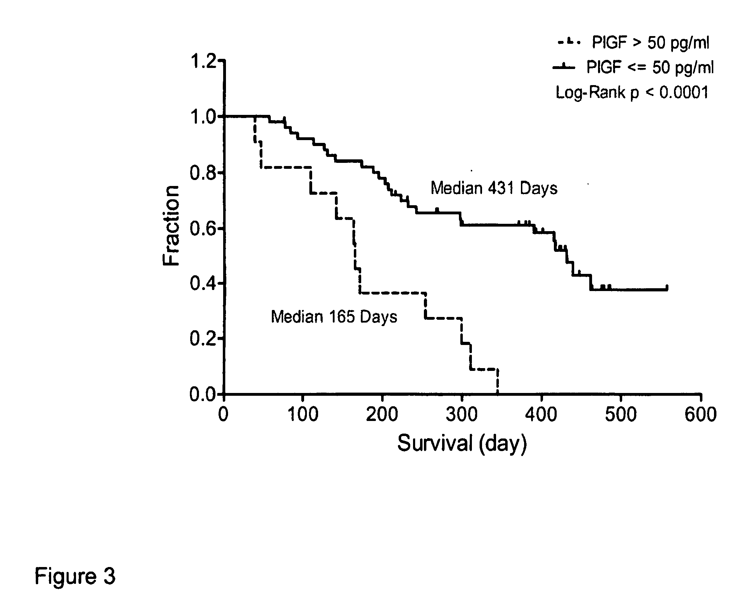

The log-rank test results indicate that plasma PlGF levels at 50 pg/ml at Day 14 of bevacizumab treatment was a significant variable that correlated with overall survival of a patient (p < 0.0003; see Figure 3). The median survival time for patients with baseline plasma PlGF levels greater than 50 pg/ml after commencing bevacizumab treatment was 165 days. In contrast, the median overall survival time for patients with plasma PlGF levels less than or equal to 50 pg/ml after commencing bevacizumab treatment was 431 days. These results indicate that plasma PlGF level in patients who have started treatment with anti-angiogenic therapy, e.g., bevacizumab, is a predictor of survival benefit for cancer patients undergoing anti-angiogenesis therapy.

## Claims

1. A method of identifying a patient suffering from cancer who may benefit from anti-cancer therapy other than or in addition to anti-angiogenic therapy, comprising
determining expression level of placental growth factor (PlGF) in a sample obtained from the patient,
wherein increased expression level of PlGF in the sample obtained from the patient as compared to a reference sample indicates that the patient may benefit from anti-cancer therapy other than or in addition to anti-angiogenic therapy.

2. A method of predicting responsiveness of a patient suffering from cancer to anti-angiogenic therapy comprising
determining expression level of PlGF in a sample obtained from the patient,
wherein increased expression level of PlGF in the sample obtained from the patient as compared to a reference sample indicates that the patient is less likely to be responsive to the anti-angiogenic therapy alone.

3. A method of monitoring effectiveness of an anti-angiogenic therapy comprising
determining expression level of PlGF in a sample obtained from the patient,
wherein increased expression level of PlGF in the sample obtained from the patient as compared to a reference sample indicates that the patient is less likely to be responsive to the anti-angiogenic therapy alone.

4. A method of optimizing therapeutic efficacy for treatment of cancer, the method comprising
determining expression level of PlGF in a sample obtained from the patient,
wherein increased expression level of PlGF in the sample obtained from the patient as compared to a reference sample indicates that the patient is less likely to be responsive to the anti-angiogenic therapy alone.

5. The method of any one of claims 1-4, wherein the expression level of PlGF in the sample obtained from the patient is increased at least about 1.5 fold as compared to a reference sample.

6. The method of any one of claims 1-4, wherein the anti-angiogenic therapy comprises administration of a VEGF antagonist.

7. The method of claim 6, wherein the VEGF antagonist is an anti-VEGF antibody.

8. The method of claim 7, wherein the anti-VEGF antibody is bevacizumab.

9. The method of claim 5, wherein the cancer is breast cancer.

10. The method of any one of claims 1-4, wherein the sample obtained from the patient is plasma.

11. The method of claim 10, wherein the expression level of PlGF is determined by detecting PlGF protein in the plasma.

12. The method of any one of claims 1-4, wherein the sample obtained from the patient is a sample obtained from the patient prior to anti-angiogenic therapy.

13. The method of any one of claims 1-4, wherein the sample obtained from the patient is a sample obtained from the patient after commencement of anti-angiogenic therapy.

14. The method of claim 12, wherein the sample obtained from the patient is a sample obtained from the patient within 24 hours after commencement of anti-angiogenic therapy.

15. The method of claim 12, wherein the sample obtained from the patient is a sample obtained from the patient within 14 days after commencement of anti-angiogenic therapy.

## Patentansprüche

1. Verfahren zum Identifizieren eines Individuums, das an Krebs leidet und von einer anderen anti-Krebsbehandlung als einer anti-angiogenetischen Behandlung oder zusätzlich zu einer anti-angiogenetischen Behandlung profitieren könnte, umfassend:
Bestimmen des Expressionsspiegels an Plazenta-Wachstumsfaktor (placental growth factor; PlGF) in einer Probe, die von dem Individuum erhalten wurde,
wobei ein erhöhter Expressionsspiegel an PlGF in der Probe, die von dem Individuum erhalten wurde, verglichen mit einer Referenzprobe, anzeigt, dass das Individuum von einer anderen anti-Krebsbehandlung als einer anti-angiogenetischen Behandlung oder zusätzlich zu einer anti-angiogenetischen Behandlung profitieren könnte.

2. Verfahren zum Prognostizieren des Ansprechens eines Individuums, das an Krebs leidet, auf eine anti-angiogenetische Behandlung, umfassend:
Bestimmen des Expressionsspiegels an PlGF in einer Probe, die von dem Individuum erhalten wurde,
wobei ein erhöhter Expressionsspiegel an PlGF in der Probe, die von dem Individuum erhalten wurde, verglichen mit einer Referenzprobe, anzeigt, dass das Individuum weniger wahrscheinlich auf die anti-angiogenetische Behandlung alleine anspricht.

3. Verfahren zur Überwachung der Wirksamkeit einer anti-angiogenetischen Behandlung, umfassend:
Bestimmen des Expressionsspiegels an PlGF in einer Probe, die von dem Individuum erhalten wurde,
wobei ein erhöhter Expressionsspiegel an PlGF in der Probe, die von dem Individuum erhalten wurde, verglichen mit einer Referenzprobe, anzeigt, dass das Individuum weniger wahrscheinlich auf die anti-angiogenetische Behandlung alleine anspricht.

4. Verfahren zum Optimieren der therapeutischen Wirksamkeit zur Behandlung von Krebs, wobei das Verfahren umfasst:
Bestimmen des Expressionsspiegels an PlGF in einer Probe, die von dem Individuum erhalten wurde,
wobei ein erhöhter Expressionsspiegel an PlGF in der Probe, die von dem Individuum erhalten wurde, verglichen mit einer Referenzprobe, anzeigt, dass das Individuum weniger wahrscheinlich auf die anti-angiogenetische Behandlung alleine anspricht.

5. Verfahren nach einem der Ansprüche 1 bis 4, wobei der Expressionsspiegel an PIGF in einer Probe, die von dem Individuum erhalten wurde, mindestens etwa 1,5fach erhöht ist im Vergleich zu einer Referenzprobe.

6. Verfahren nach einem der Ansprüche 1 bis 4, wobei die anti-angiogenetische Behandlung das Verabreichen eines VEGF-Antagonisten umfasst.

7. Verfahren nach Anspruch 6, wobei der VEGF-Antagonist ein anti-VEGF-Antikörper ist.

8. Verfahren nach Anspruch 7, wobei der anti-VEGF-Antikörper Bevacizumab ist.

9. Verfahren nach Anspruch 5, wobei der Krebs Brustkrebs ist.

10. Verfahren nach einem der Ansprüche 1 bis 4, wobei die Probe, die von dem Individuum erhalten wurde, Plasma ist.

11. Verfahren nach Anspruch 10, wobei der Expressionsspiegel an PlGF durch Nachweisen des PlGF-Proteins im Plasma bestimmt wird.

12. Verfahren nach einem der Ansprüche 1 bis 4, wobei die Probe, die von dem Individuum erhalten wurde, eine Probe ist, die von dem Individuum vor der anti-angiogenetischen Behandlung erhalten wurde.

13. Verfahren nach einem der Ansprüche 1 bis 4, wobei die Probe, die von dem Individuum erhalten wurde, eine Probe ist, die von dem Individuum nach Beginn der anti-angiogenetischen Behandlung erhalten wurde.

14. Verfahren nach Anspruch 12, wobei die Probe, die von dem Individuum erhalten wurde, eine Probe ist, die von dem Individuum innerhalb von 24 Stunden nach Beginn der anti-angiogenetischen Behandlung erhalten wurde.

15. Verfahren nach Anspruch 12, wobei die Probe, die von dem Individuum erhalten wurde, eine Probe ist, die von dem Individuum innerhalb von 14 Tagen nach Beginn der anti-angiogenetischen Behandlung erhalten wurde.

## Revendications

1. Procédé d'identification d'un patient souffrant du cancer qui peut bénéficier d'une thérapie anticancéreuse autre qu'une thérapie anti-angiogénique ou en plus d'une thérapie anti-angiogénique, comprenant
la détermination du niveau d'expression du facteur de croissance placentaire (PlGF) dans un échantillon prélevé chez le patient,
dans lequel un niveau d'expression accru du PlGF dans l'échantillon prélevé chez le patient comparé à un échantillon de référence indique que le patient peut bénéficier d'une thérapie anticancéreuse autre qu'une thérapie anti-angiogénique ou en plus d'une thérapie anti-angiogénique.

2. Procédé de prédiction de la réponse d'un patient souffrant du cancer à une thérapie anti-angiogénique comprenant
la détermination du niveau d'expression du PlGF dans un échantillon prélevé chez le patient, dans lequel un niveau d'expression accru du PlGF dans l'échantillon prélevé chez le patient comparé à un échantillon de référence indique que le patient est moins susceptible de répondre à la thérapie anti-angiogénique seule.

3. Procédé de surveillance de l'efficacité d'une thérapie anti-angiogénique comprenant
la détermination du niveau d'expression du PlGF dans un échantillon prélevé chez le patient, dans lequel un niveau d'expression accru du PlGF dans l'échantillon prélevé chez le patient comparé à un échantillon de référence indique que le patient est moins susceptible de répondre à la thérapie anti-angiogénique seule.

4. Procédé d'optimisation de l'efficacité thérapeutique du traitement du cancer, le procédé comprenant
la détermination du niveau d'expression du PlGF dans un échantillon prélevé chez le patient, dans lequel un niveau d'expression accru du PlGF dans l'échantillon prélevé chez le patient comparé à un échantillon de référence indique que le patient est moins susceptible de répondre à la thérapie anti-angiogénique seule.

5. Procédé selon l'une quelconque des revendications 1 à 4, dans lequel le niveau d'expression du PlGF dans l'échantillon prélevé chez le patient est accru d'au moins environ 1,5 fois comparé à un échantillon de référence.

6. Procédé selon l'une quelconque des revendications 1 à 4, dans lequel la thérapie anti-angiogénique comprend l'administration d'un antagoniste du VEGF.

7. Procédé selon la revendication 6, dans lequel l'antagoniste du VEGF est un anticorps anti-VEGF.

8. Procédé selon la revendication 7, dans lequel l'anticorps anti-VEGF est le bévacizumab.

9. Procédé selon la revendication 5, dans lequel le cancer est le cancer du sein.

10. Procédé selon l'une quelconque des revendications 1 à 4, dans lequel l'échantillon prélevé chez le patient est le plasma.

11. Procédé selon la revendication 10, dans lequel le niveau d'expression du PlGF est déterminé par détection de la protéine PlGF dans le plasma.

12. Procédé selon l'une quelconque des revendications 1 à 4, dans lequel l'échantillon prélevé chez le patient est un échantillon prélevé chez le patient avant la thérapie anti-angiogénique.

13. Procédé selon l'une quelconque des revendications 1 à 4, dans lequel l'échantillon prélevé chez le patient est un échantillon prélevé chez le patient après le début de la thérapie anti-angiogénique.

14. Procédé selon la revendication 12, dans lequel l'échantillon prélevé chez le patient est un échantillon prélevé chez le patient dans les 24 heures qui suivent le début de la thérapie anti-angiogénique.

15. Procédé selon la revendication 12, dans lequel l'échantillon prélevé chez le patient est un échantillon prélevé chez le patient dans les 14 jours qui suivent le début de la thérapie anti-angiogénique.
